# EUROPÄISCHE PATENTANMELDUNG

(11) **EP 0 590 219 A1**
(43) Veröffentlichungstag der Anmeldung: **06.04.1994**
(21) Anmeldenummer: 92810742.4
(22) Anmeldetag: 02.10.1992
(51) Int. Cl.: C12M 3/08, B02C 19/12, G01N 1/28

(54) **Verfahren zum Zerkleinern von weichem Gewebe und Mittel zum Durchführen des Verfahrens**

(71) Anmelder: SULZER Medizinaltechnik AG, CH-8404 Winterthur (CH)
(72) Erfinder: Bittmann, Peter, Dr., CH-8057 Zürich (CH); Nadler, Daniel, CH-8355 Aadorf (CH); Müller-Glauser, Werner, Dr., CH-8542 Wiesendangen (CH)
(74) Vertreter: Hammer, Bruno, Dr.

(57) **Zusammenfassung**

Das Verfahren zum Zerkleinern von weichem Gewebe (4) aus tierischem oder menschlichem Körper wird mit einer Vorrichtung (10) durchgeführt, welche einen Vorratsbehälter (1), ein Schneidorgan (2) sowie einen Auffangsbehälter (3) aufweist und welche einen für Keime undurchlässigen Abschluss gegen die Umgebung bildet. Das Gewebe wird in einer sterilen Umgebung dem Körper entnommmen und in den Vorratsbehälter der geöffneten Vorrichtung gefüllt. Nachdem der Vorratsbehälter zusammen mit dem Schneidorgan und dem Auffangsbehälter zu einer betriebsbereiten Vorrichtung zusammengesetzt worden ist, wird unter Verwendung von druckausübenden Mitteln, vorzugsweise Druckgas (11'), das Gewebe aus dem Vorratsbehälter in den Auffangsbehälter gefördert, wobei gleichzeitig das Gewebe durch das Schneidorgan zerkleinert wird.

## Beschreibung

Die Erfindung betrifft ein Verfahren zum Zerkleinern von weichem Gewebe aus tierischem oder menschlichem Körper sowie Mittel zum Durchführen des Verfahrens.

Es ist bekannt, bei der Implantation von Blutgefäss-Prothesen, beispielsweise für einen Arterienersatz, Hybridprothesen zu verwenden. Eine derartige Gefässprothese besteht aus einer schlauchartigen, porösen Kunststoffwand, deren innere Oberfläche mit körpereigenen Endothelzellen beladen ist. Die Zellen können aus Fettgewebe des Patienten, das mikrovaskuläre Endothelzellen enthält, gewonnen werden (siehe B.E.Jarell et al. "Use of an endothelial monolayer on a vascular graft prior to implantation", Ann.Surg.1986;203:671-678). Die Präparation der Hybridprothese, d.h. das Gewinnen der Zellen sowie das Beschichten des Kunststoffschlauchs, muss während der Operation, bei welcher der Gefässersatz erfolgt, ausgeführt werden; für die Präparation steht höchstens eine Stunde zur Verfügung.

Der Chirurg entnimmt das Fettgewebe in Stücken, die rund 20 - 50 g wiegen. Anschliessend werden die Gewebestücke zerkleinert, sodass Gewebeteilchen mit einem Gewicht von rund 0.03 - 0.1 g entstehen. Mittels eines enzymatischen Aufschlusses, bei dem das Bindegewebe teilweise abgebaut wird, lassen sich die Zellen freisetzen; sie müssen anschliessend in einem weiteren Präparationsschritt noch vom Fettanteil abgetrennt werden.

Das Zerkleinern der Gewebestücke muss schonend durch ein sorgfältiges Schneiden geschehen, bei dem möglichst viele Zellen unversehrt bleiben. Bei den bekannten Verfahren wird das Zerkleinern der groben Gewebestücke in kleine Gewebeteilchen durch aufwendige Handarbeit mittels Skalpell ausgeführt. Diese Tätigkeit ist zusätzlich erschwert, da Sterilität erforderlich ist. Es ist Aufgabe der Erfindung, ein Verfahren zu schaffen, mit welchem ein schnelles und schonendes Zerkleinern von Gewebe aus menschlichen oder tierischen Weichteilen - unter Einhaltung von sterilen Bedingungen - durchführbar ist. Diese Aufgabe wird durch die in Anspruch 1 genannten Massnahmen gelöst. Dabei wird eine Anlage verwendet, die beispielsweise durch die Merkmale des unabhängigen Anspruchs 3 gekennzeichnet ist. Mehrere Ausführungsformen der erfindungsgemässen Anlage, welche insbesondere verschiedene Schneidorgane aufweisen, sind durch die abhängigen Ansprüche 4 bis 12 gekennzeichnet.

Die im Anspruch 1 genannten druckausübenden Mitteln bestehen vorzugsweise aus einem Druckgas, mit dem unter gleichzeitiger Nutzung der Schwerkraft das Gewebe durch das Schneidorgan getrieben wird. Es ist auch denkbar, das Gewebe mittels einer Gewindespindel und einem Kolben zu fördern. Weitere Möglichkeiten bestehen darin, für das kraftübermittelnde Medium statt Gas eine Flüssigkeit zu verwenden, wobei die Trennung zwischen Gewebe und Flüssigkeit mittels eines Kolbens oder eines Balgs hergestellt werden kann.

Das erfindungsgemässe Verfahren, das ursprünglich für das Zerkleinern von Fettgewebe entwickelt worden ist, lässt sich auch für andere weiche, nicht-knöcherne Gewebe verwenden. Das Zerkleinern von Lebergewebe kann als Beispiel genannt werden. Es ist ein Verfahren zur Blutwäsche bekannt, bei dem mittels freigesetzten und auf ein Substrat übertragenen Schweineleberzellen Blut von Patienten mit ungenügender Leberfunktion entgiftet wird.

Nachfolgend wird die Erfindung im Zusammenhang mit den Zeichnungen erläutert. Es zeigen:
- Fig. 1: eine schematische Darstellung einer einfachen Ausführungsform der erfindungsgemässen Anlage,
- Fig. 2: den aus der Anlage der Fig.1 entnommenen Vorratsbehälter,
- Fig. 3: einen Längsschnitt durch eine zweite Ausführungsform der erfindungsgemässen Zerkleinerungsvorrichtung, welche ein fleischwolfartiges Schneidorgan aufweist,
- Fig. 4: eine Ansicht der Anlage mit der Ausführungsform gemäss Fig.3,
- Fig. 5: ein Schneidorgan für die Ausführungsform der Fig.1,
- Fig. 6: Teile eines fleischwolfartigen Schneidorgans,
- Fig. 7: ein Messer für ein fleischwolfartiges Schneidorgan,
- Fig.8a-c: Querschnitte durch Messer für fleischwolfartige Schneidorgane und
- Fig.9a,b: eine besondere Ausführungsform des fleischwolfartigen Schneidorgans.

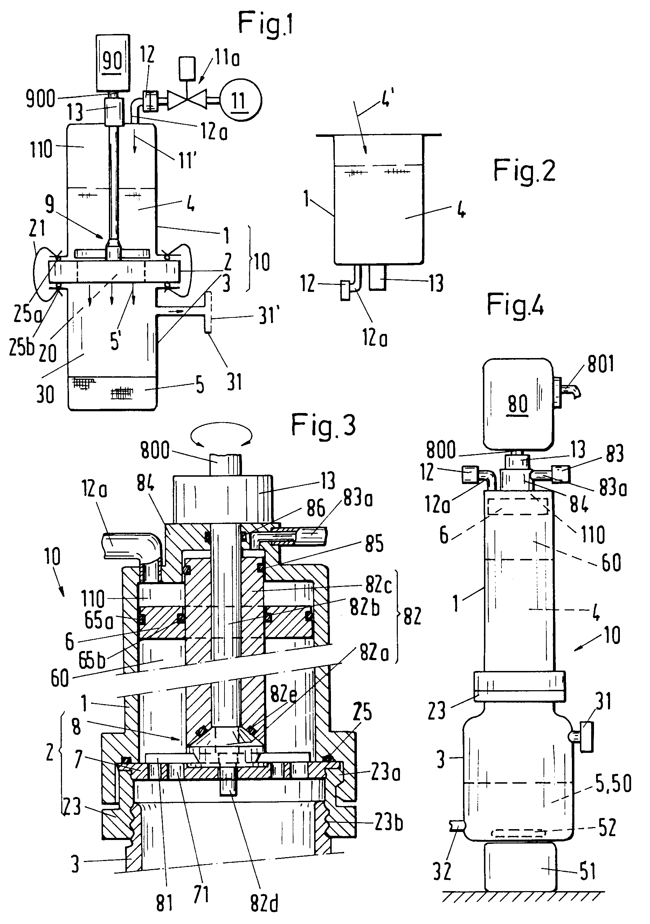

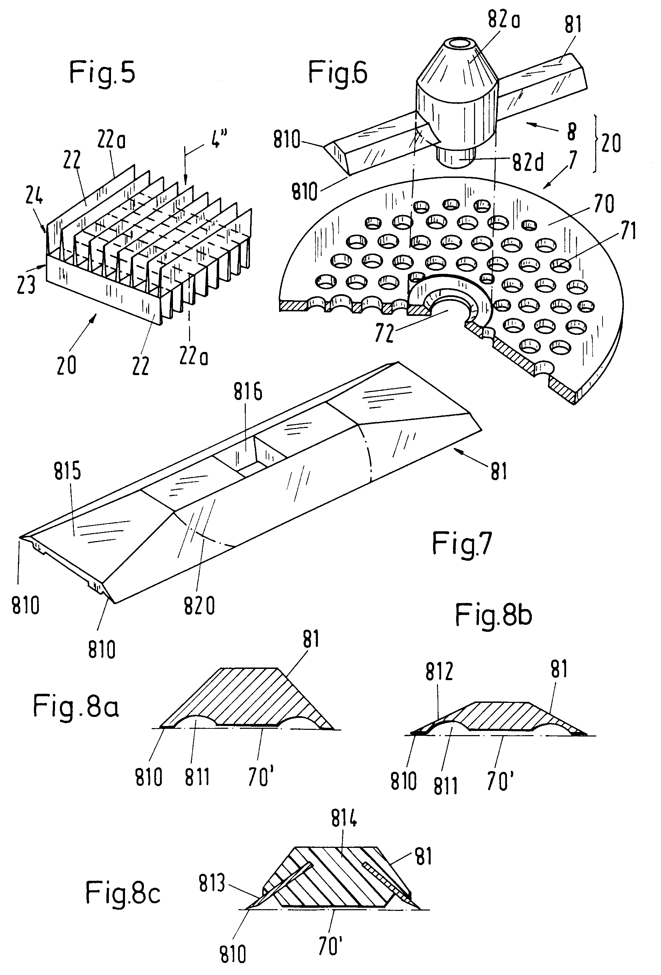

Die erfindungsgemässe Anlage der Fig.1 besteht aus der Vorrichtung 10, die sich aus dem Vorratsbehälter 1, dem Schneidorgan 2 und dem Auffangbehälter 3 zusammensetzt, sowie der Druckgasquelle 11. Der Vorratsbehälter 1 lässt sich aus der Anlage entfernen, sodass - siehe Fig.2 - das zu behandelnde Gewebe 4 in dieses Gefäss 1 gebracht werden kann (Pfeil 4'). Für das Schneidorgan 2 ist ein Schneidmittel 20 vorgesehen, wie es als Schrägbild in Fig.5 gezeigt ist. Eine zweite Ausführungsform eines möglichen Schneidmittels 20 ist in Fig.6 als Explosionszeichnung dargestellt; es bestehen Ähnlichkeiten mit den Schneidmitteln eines Fleischwolfs. Bei der Vorrichtung 10 der Figuren 3 und 4 ist ein solches fleischwolfartiges Schneidorgan 2 vorgesehen. In der betriebsbereiten Anlage ist der das Gewebe 4 enthaltende Behälter 1 über dem Schneidorgan 2 angeordnet, sodass das Gewebe aufgrund der Schwerkraft auf dem Schneidmittel 20 aufliegt.

Die Vorrichtung 10 der Fig.1 lässt sich mittels Klammern 21 und Dichtungsringen 25a, 25b aus den drei Teilen 1, 2 und 3 zu einer Einheit zusammensetzen, die für das zu behandelnde Gewebe 4 und das zerkleinerte Gewebe 5 eine für Keime undurchlässige Hülle bildet. Bei der Vorrichtung 10 der Fig.3 wird die entsprechende Verbindung durch einen Zwischenring 23 hergestellt, der Teil des Schneidorgans 2 ist und mit dem Vorratsbehälter 1 über einen Bajonettverschluss 23a und mit dem Auffangbehälter 3 über eine Verschraubung 23b verbindbar ist. Zur Abdichtung der Stossstelle zwischen Behälter 1 und Schneidorgan 2 ist ein Dichtungsring 25 vorgesehen. Es sind selbstverständlich auch andere Verbindungsmöglichkeiten denkbar.

Das Gewebe 4 bildet dank seiner Weichheit im Vorratsbehälter 1 der zusammengesetzten Vorrichtung 10 einen gasdichten Abschluss zwischen einem Gasraum 110 und dem Schneidmittel 20. Diesem Gasraum 110 wird durch die Druckgasquelle 11 über das einstellbare Drosselorgan 11a und die lösbare Kuppelstelle 12 Druckgas 11' zugeführt; das Gewebe 4 wird dadurch bei gleichzeitiger Zerkleinerung durch das Schneidmittel 20 gepresst und in den Auffangsbehälter 3 gefördert (Pfeile 5'). Damit dort im Luftraum 30 durch die Zufuhr des zerkleinerten Gewebes 5 nicht ein Überdruck entsteht, ist am Auffangsbehälter 3 ein durckausgleichendes Luftablasselement 31 mit einer feinporigen Auslasszone 31' - bestehend beispielsweise aus einem Sterilfilter - vorgesehen. Das Druckgas 11' muss steril und frei von sonstigen Verunreinigungen sein. Im Vorratsbehälter 1 kann ein langsam drehender Rührer 9 dazu dienen, einen Gasdurchbruch vom Gasraum 110 in den Luftraum 30 unterhalb des Schneidorgans 2 zu verhindern - zumindest solange dazu die Menge des Gewebes 4 ausreicht. Über eine Kuppelstelle 13 ist der Rührer 9 an die Antriebswelle 90a eines Motors 90 anschliessbar. Die Kuppelstelle 13 muss wie die Kuppelstelle 12 lösbar ausgebildet sein, damit der Vorratsbehälter 1 aus der Vorrichtung 10 entfernt und zum Einfüllen des Gewebes 4 (Fig.2) so gehalten werden kann, dass die Öffnung nach oben zu liegen kommt.

Damit im Auffangsbehälter 3 kein den Schneidvorgang behindernder Überdruck aufgebaut wird, kann statt der feinporigen Auslasszone 31' des Luftablasselements 31 beispielsweise auch ein leichtdehnbarer Gummiballon vorgesehen werden. Es ist auch denkbar, den Auffangsbehälter 3 derart auszubilden, dass er ein variables Volumen annehmen kann, wobei eine Volumenvergrösserung durch einen geringen Druckanstieg bewirkbar sein soll.

Mit Vorteil wird im Vorratsbehälter 1 ein Kolben 6 vorgesehen (Fig.3), der einen beweglichen aber gasdichten Abschluss (Dichtungen 65a, 65b) zwischen dem Gasraum 110 und einem in Kontakt mit dem Gewebe 4 stehenden Luftraum 60 bildet (siehe auch Fig.4). Damit ist die Sterilität des Druckgases 11' nicht mehr erforderlich, und die Gefahr eines Gasdurchbruchs zwischen Gasraum 110 und Luftraum 30 besteht auch nicht mehr. Es ist auch denkbar, anstelle des Kolbens eine balgartige Trenneinrichtung zwischen Gasraum 110 und Luftraum 60 vorzusehen.

Das in Fig.5 dargestellte Schneidmittel 20 besteht aus Schneidlamellen 22 mit Schneidkanten 22a, welche mit zwei im rechten Winkel quer zu einander ausgerichteten Gittern 23 und 24 einen Doppelrost bilden. Mit dem Pfeil 4'' ist die Transportrichtung des dem Schneidorgan zugeführten Gewebes angegeben. Der zwischen den beiden Gitterrichtungen liegende Winkel kann auch von 90° verschieden sein.

Das Schneidmittel 20 des fleischwolfartigen Schneidorgans, das in Fig.6 zu sehen ist, setzt sich aus einer perforierten Platte 7 und einer beweglichen Komponente 8 mit messerartigen Schneidelementen 81 zusammen. Die Schneidkanten 810 dieser Messer 81 sind auf der Gewebeeintrittsseite 70 über die Platte 7 bewegbar. Die Oberfläche 70 ist eben und sehr glatt ausgebildet, und die Löcher 71 weisen Eintrittsöffnungen mit scharfkantigen Rändern auf. Das Gewebe wird dadurch zerkleinert, dass das in die Löcher 71 gepresste Gewebe zwischen den Messerschneiden 810 und den Löcherkanten abgeschert wird. Der Lochdurchmesser kann zwischen 1 und 6 mm liegen; vorzugsweise liegt er im Bereich zwischen 2 und 4 mm.

Bei dem anhand der Figuren 3, 4 und 6 beschriebenen Ausführungsbeispiels ist der Vorratsbehälter 1 zylindrisch und die bewegliche Komponente 8 als drehbares Element ausgebildet: Die Schneidelemente 81 sind radiale, messerartige Flügel an einer zylindrischen Welle 82, deren Mittellinie mit jener des Vorratsbehälters 1 zumindest angenähert zusammenfällt; die Welle 82 ist über eine lösbare Kuppelstelle 13 mit einer Antriebswelle 800 eines Motors 80 verbunden (Energiezufuhr über die Leitung 801). Die Welle 82 besteht gemäss Fig.3 aus einem Messerhalter 82a, einer zentralen Stange 82b, einem Mantelrohr 82c, einem Lagerzapfen 82d, für den in der Platte 7 ein zentrale Bohrung 72 (siehe Fig.6) vorgesehen ist, und ferner einem Dichtungsring 82e. Der ringförmig ausgebildete Kolben 6 umschliesst die Welle 82 dicht. Der obere Teil der Welle 82 ist auch als Kolben (Dichtungsring 85) ausgebildet, der in das Zylindergehäuse 84 ragt. Der Durchgang der Stange 82b durch die Wand des Zylindergehäuses 84 ist mit einem Dichtungsring 86 abgedichtet.

Mit einer zweiten Druckgasquelle (nicht dargestellt), für die eine lösbare Kuppelstelle 83 (Fig.4) und eine Leitung 83a vorgesehen sind, lassen sich über die Welle 82 die Messer 81 pneumatisch auf die perforierte Platte 7 aufpressen. Die pneumatisch erzeugte Anpresskraft kann bei gleichem Druck des Druckgases mittels konstruktiver Massnahmen, nämlich durch eine Vergrösserung der druckbeaufschlagten Fläche und eine entsprechende Vergrösserung des Zylindergehäuses 84, angehoben werden. Wie experimentell festgestellt worden ist, muss die Anpresskraft mindestens rund 2500 N/m - bezogen auf die gesamte Länge der Schneidkanten 810 - betragen.

Damit möglichst viel des zu behandelnden Gewebes in den Arbeitsbereich des Schneidmittels 20 gelangen kann, muss das Messer 81 flach ausgebildet sein. Es muss aus einem biegesteifen Material bestehen, das erlaubt, die benötigten Anpresskräfte gleichmässig über die ganze Länge der Schneidkanten 810 auf die Oberfläche 70 einwirken zu lassen. Der Einfachheit halber weist das Messer 81 einen konstanten Querschnitt auf, wie es in Fig.6 der Fall ist. Es ist aber auch möglich, dem Messer 81 einen radial nach aussen sich verjüngenden Querschnitt zu geben. Ein derartiges Messer 81 ist in Fig.7 dargestellt: An der Messeroberseite, zwischen der Wellenoberfläche, die als strichpunktierte Kurve 820 angedeutet ist, und den äusseren Kanten des Messers sind schräg verlaufende Flächen 815 vorgesehen. (Der quadratische Durchbruch 816 im Zentrum des Messers ist für den Eingriff eines Vierkants vorgesehen, über den das Drehmoment der Welle 82 auf das Messer 81 übertragen wird.)

In den Figuren 8a bis 8c sind anhand von Querschnitten weitere Beispiele des Schneidelements 81 gezeigt. Mit der strichpunktierten Linie 70' ist die Lage der Plattenoberfläche 70 angegeben. Parallel zur Schneidkante 810 sind Nuten 811 vorgesehen, die das Schleifen der Kante erleichtern. Beim flachen Messer 81 der Fig.8b ergibt sich durch die Nut 811 ein dünne Stelle 812 hinter der Schneidkante 810, mit der bei geeigneter Materialwahl eine federnde Nachgiebigkeit der Schneidkante erzeugbar ist. Fig.8c zeigt ein Messer 81, bei dem Schneidlamellen 813 in einen Grundkörper 814 aus Kunststoff eingegossen sind.

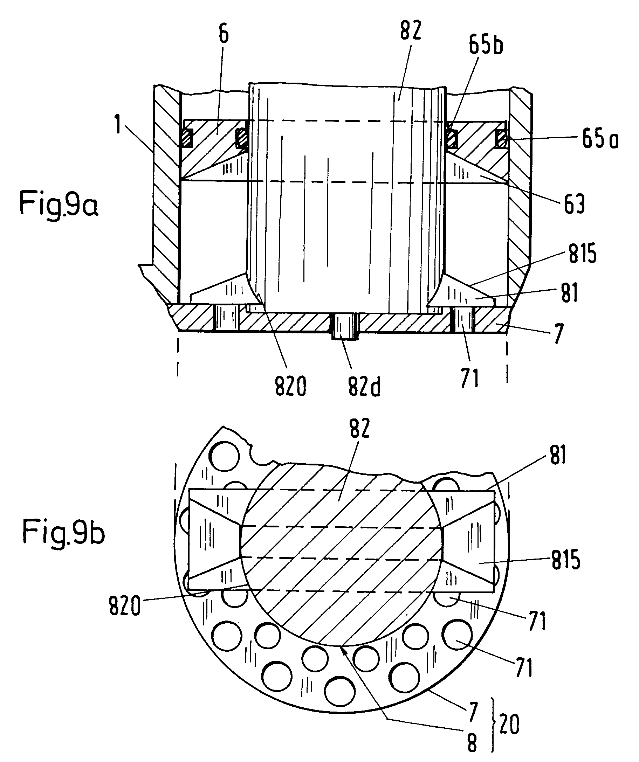

Das in Fig.9a,b dargestellte Scheidorgan weist ein Messer 81 der in Fig.7 gezeigten Art auf. Entsprechend ist beim Kolben 6 eine konisch ausgebildete Unterseite 63 vorgesehen. Bei dieser Formgebung entsteht ein kleinerer Rest an Gewebe, das nicht zerkleinert werden kann, als bei der Vorrichung gemäss Fig.3, wo die Kolbenunterseite parallel zur Plattenoberfläche 70 verläuft und das Messer 81 einen konstanten Querschnitt aufweist. Die Wahl eines grossen Durchmessers der Welle 82, zufolge welcher der gelochte ringförmige Teil der Platte 7 wesentlich kleiner als bei den Abmessungen gemäss Fig.6 ausfällt, trägt dazu bei, dass der nicht zerkleinerbare Teil des Gewebes weiter verringert wird.

Die ringförmigen Kolben 6 in den Figuren 3 und 9a weisen je einen inneren Dichtungsring 65b und einen äusseren 65a auf. Um eine bessere Abdichtung zwischen den Räumen 110 und 60 (Fig.3) sowie eine bessere Gleitfähigkeit des Kolbens 6 zu erhalten, können jeweils zwei Dichtungsringe vorgesehen werden.

Beim Zerkleinern von Fettgewebe mittels eines fleischwolfartigen Schneidorgans 2 mit rotierenden Schneidelementen 81 haben sich Probleme mit Kollagenfasern ergeben. Diese Fasern, die teilweise unzerschnitten bleiben, behindern den Transport des zerkleinerten Gewebes 5 durch die Lochplatte 7 und beeinträchtigen den Schneidvorgang. Zum Teil wickeln sich auch Fasern an der Welle 82 auf. Indem mit einer relativ grossen Kraft die Schneidelemente 81 auf die Lochplatte 7 aufgepresst werden und indem der Drehsinn periodisch umgekehrt wird - jeweils nach einer Drehung um rund 180° - lässt sich die Beeinträchtigung des erfindungsgemässen Verfahrens beheben. Damit die Bewegungsumkehr möglich ist, sind die Schneidelemente 81 mit zwei Schneiden 810 ausgebildet, wie es in den Figuren 6 bis 8c gezeigt ist. Es ist selbstverständlich auch ein Antriebsmotor 80 (Fig.4) vorzusehen, mit dem die vorteilhafte Hin- und Herbewegung der Schneidelemente 81 ausführbar ist.

Wie eingangs erwähnt worden ist, kann auf die mechanische Zerkleinerung des Gewebes 4 ein enzymatischer Aufschluss folgen, bei dem das zerkleinerte Geweben 5 desintegriert wird und einzelne Zellen freigesetzt werden. Mit Vorteil wird der Auffangsbehälter 3 zur Durchführung dieses Aufschlusses genutzt (siehe Fig.4): das zerkleinerte Gewebe 5 wird im Auffangsbehälter 3 mit einer Lösung 50 eines Enzyms - beispielsweise Collagenase - in Kontakt gebracht. Dabei sorgt ein magnetisches Rührelement 52 mit einem Antrieb 51 für eine Durchmischung von Enzymlösung und Gewebe. Nach erfolgtem Aufschluss kann das Gemisch 5, 50 über einen Auslass 32 für weitere Verfahrensschritte (Trennen der Zellen von Fett und Enzym) aus dem Auffangsbehälter 3 entfernt werden.

## Patentansprüche

1. Verfahren zum Zerkleinern von weichem Gewebe aus tierischem oder menschlichem Körper, bei welchem Verfahren das Gewebe nach der Entnahme aus dem Körper in einer Vorrichtung behandelt wird, welche einen Vorratsbehälter, ein Schneidorgan sowie einen Auffangsbehälter aufweist und welche einen für Keime undurchlässigen Abschluss gegen die Umgebung bildet, wobei
a) das Gewebe in einer sterilen Umgebung dem Körper entnommmen und in den Vorratsbehälter der geöffneten Vorrichtung gefüllt wird,
b) der Vorratsbehälter zusammen mit dem Schneidorgan und dem Auffangsbehälter zu einer betriebsbereiten Vorrichtung zusammengesetzt wird und
c) das Gewebe unter Verwendung von druckausübenden Mitteln aus dem Vorratsbehälter durch das Schneidorgan bei gleichzeitiger Zerkleinerung des Gewebes in den Auffangsbehälter gefördert wird.

2. Verfahren nach Anspruch 1, dadurch gekennzeichnet, dass das zerkleinerte Gewebe im Auffangsbehälter mit einer Enzymlösung in Kontakt gebracht wird.

3. Anlage zum Durchführen des Verfahrens nach Anspruch 1 oder 2, mit einer Vorrichtung (10), die einen Vorratsbehälter (1), ein Schneidorgan (2), einen Auffangsbehälter (3) und Mittel (21,25a,25b) zum dichten Zusammensetzen der drei genannten Vorrichtungsteile (1,2,3) umfasst, mit einer Druckgasquelle (11), mit einer lösbaren Kuppelstelle (12) für eine Druckgasleitung (12a) am Vorratsbehälter (1), mit einem einstellbaren Drosselorgan (11a) zwischen Druckgasquelle und Vorratsbehälter und mit einem druckausgleichenden Luftablasselement (31) am Auffangsbehälter (3).

4. Anlage nach Anspruch 3, dadurch gekennzeichnet, dass der Vorratsbehälter (1) zylindrisch ausgebildet ist und einen axial verschiebbaren Kolben (6) aufweist, der den Vorratsbehälter in einen Teilraum (60) für das Gewebe (4) und einen Teilraum (110) für das Druckgas (11') unterteilt.

5. Anlage nach einem der Ansprüche 3 oder 4, dadurch gekennzeichnet, dass das Schneidorgan (2) einen Doppelrost (23,24) mit Schneidlamellen (22), die zwei quer zu einander ausgerichtete Gitter bilden, aufweist.

6. Anlage nach Anspruch 5, dadurch gekennzeichnet, dass ein Rührorgan (9) im Vorratsbehälter (1) vorgesehen ist, welches über eine lösbare Kuppelstelle (13) mit einem Antriebsmotor (90) verbunden ist.

7. Anlage nach einem der Ansprüche 3 oder 4, dadurch gekennzeichnet, dass das Schneidorgan (20) eine perforierte Platte (7) und Schneidelemente (81) aufweist, die mit den Schneidkanten (810) auf der Gewebeeintrittsseite (70) über die Platte bewegbar sind.

8. Anlage nach Anspruch 7, dadurch gekennzeichnet, dass der Vorratsbehälter (1) zylindrisch ist, die Schneidelemente (81) als radiale, messerartige Flügel an einer zylindrischen Welle (82), deren Mittellinie mit jener des Vorratsgefässes zumindest angenähert zusammenfällt, ausgebildet sind und die Welle über eine lösbare Kuppelstelle (13) mit einem Antriebsmotor (80) verbunden ist.

9. Anlage nach Anspruch 7 oder 8, dadurch gekennzeichnet, dass die Schneidelemente (81) pneumatisch mittels einer zweiten Druckgasquelle und über die Welle (82) auf die perforierte Platte (7) anpressbar sind.

10. Anlage nach Anspruch 7 oder 8, dadurch gekennzeichnet, dass mit dem Antriebsmotor (80) eine Hin- und Herbewegung der Schneidelemente (81) ausführbar ist.

11. Anlage nach einem der Ansprüche 8 bis 10, dadurch gekennzeichnet, dass die Löcher (71) der Platte (7) in einer ringförmigen Zone angeordnet sind und dass der innere Ringdurchmesser zumindest angenähert gleich gross wie der Wellendurchmesser ist.

12. Anlage nach einem der Ansprüche 7 bis 11, dadurch gekennzeichnet, dass die Durchmesser der Plattenlöcher (71) im Bereich zwischen 1 und 6 mm, vorzugsweise zwischen 2 und 4 mm, liegen.

13. Verwendung des Verfahrens nach Anspruch 1 oder 2, mit einer Anlage nach einem der Ansprüche 3 bis 12, zum Bereitstellen von Endothelzellen aus Fettgewebe für die Beladung der Innenseite von Blutgefässprothesen.

14. Verwendung des Verfahrens nach Anspruch 1 oder 2, mit einer Anlage nach einem der Ansprüche 3 bis 12, zum Bereitstellen von Leberzellen aus Schweinelebern für die Blutwäsche von Patienten mit ungenügender Leberfunktion.
